# EUROPEAN PATENT APPLICATION

(11) **EP 4 177 339 A1**
(43) Date of publication of application: **10.05.2023**
(21) Application number: 21833040.5
(22) Date of filing: 01.07.2021
(51) Int. Cl.: C12N 5/077, C08F 220/04, C08F 220/34, C12M 3/00, C12N 5/0735, C12N 5/10

(54) **METHOD FOR PRODUCING CARTILAGE TISSUE**

(30) Priority: 03.07.2020 JP 2020115594
(71) Applicant: National University Corporation Okayama University, Kita-ku, Okayama-shi, Okayama 700-8530 (JP); Kake Educational Institution, Okayama-shi, Okayama 700-0005 (JP); Nissan Chemical Corporation, Tokyo 103-6119 (JP)
(72) Inventor: TAKARADA Takeshi, Okayama-shi, Okayama 700-8530 (JP); IWAI Ryosuke, Okayama-shi, Okayama 700-0005 (JP); SUZUKI Kohei, Funabashi-shi, Chiba 274-0052 (JP); FUKASAWA Natsuki, Shiraoka-shi, Saitama 349-0294 (JP)
(74) Representative: dompatent von Kreisler Selting Werner - Partnerschaft von Patent- und Rechtsanwälten mbB
(86) International application number: PCT/JP2021/024967
(87) International publication number: WO 2022/004846

(57) **Abstract**

The present invention is to provide a method for producing a cartilage tissue which comprises a step of providing a substrate for producing cell aggregates provided with a plurality of spots comprising a copolymer containing recurring units derived from monomers represented by the following formulae (I) and (II): 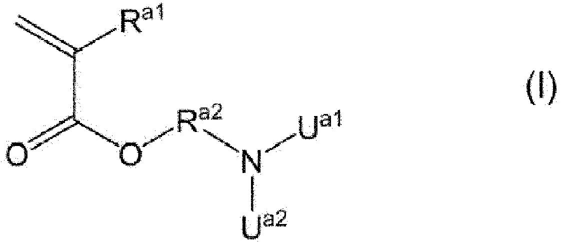 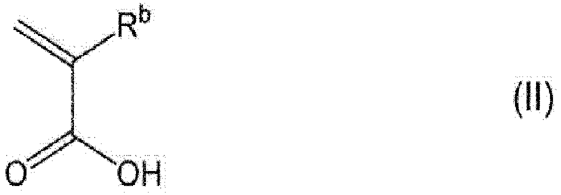 [wherein U^{a1}, U^{a}, R^{a1}, R^{a2} and R^{b} are as described in the specification and claims] on a substrate having an ability to suppress adhesion of cells; a step of seeding human cartilage progenitor cells which are positive for PRRX1 protein and derived from pluripotent stem cells on the substrate; a step of producing cell aggregates by culturing the cells; and a step of culturing the aggregates to produce a cartilage tissue.

## Description

### TECHNICAL FIELD

The present invention relates to a method for producing cartilage tissue.

### BACKGROUND ART

In the method of transplanting autologous cultured cartilage tissue, which is one of the treatment for articular cartilage damage, patient's burden is heavy since two operations of cartilage collection and cultured cartilage transplantation are required. From the viewpoint of reducing the patient's burden, etc., it has been proposed various kinds of regenerative medical technologies for producing cartilage tissue for transplantation not from autologous culture but from differentiation induction and culture of various kinds of stem cells. For example, it has been reported a technique to produce vitreous cartilage tissue by inducing induced pluripotent stem cells (iPS cells) in mesoderm, and then culturing in the presence of ascorbic acid, BMP2, TGFβ and GDF5, part of the cells on the dish form cartilage cell aggregates, and peeling and culturing the same in suspension (for example, see Non-Patent Document 1).

### PRIOR ART DOCUMENTS

### NON-PATENT DOCUMENT

Non-Patent Document 1: A. Yamashita, M. Morioka, Y. Yahara, M. Okada, T. Kobayashi, S. Kuriyama, S. Matsuda, N. Tsumaki, Generation of scaffoldless hyaline cartilaginous tissue from human iPSCs, Stem cell reports 4 (2015) 404-418

### SUMMARY OF THE INVENTION

### PROBLEMS TO BE SOLVED BY THE INVENTION

According to the above-mentioned technique, vitreous cartilage tissue as a final product can be produced from human pluripotent stem cells, but intermediate stage cells (= human cartilage progenitor cells) are not specified. Therefore, there was a problem that it was difficult to mass-produce cartilage regenerating materials with high efficiency and uniform tissue shape and properties.

### MEANS TO SOLVE THE PROBLEMS

The present inventors have been diligently studying cells at an intermediate stage in the production of cartilage tissue from stem cells and a method for producing the same, and a method for producing cartilage tissue from the cells, and have also filed patent applications for some of these (for example, see PCT/JP2020/035517). In this method, cells having an intermediate stage differentiation orientation (that is, cartilage progenitor cells) can be expansively cultured and stocked, and since the same lot of quality-controlled cells can be treated to induce cartilage differentiation to make it into cartilage tissue, and the produced cartilage tissue does not contain cells other than cartilage cells, it is advantageous for using cartilage tissue for use in regenerative medicine. The present invention was completed by finding that by producing cartilage tissue from such cartilage progenitor cells under prescribed culture conditions, it is possible to produce large cartilage tissue pieces from a small number of cells as well as efficient cell growth.

The present invention includes the following.
[1] A method for producing cartilage tissue which comprises a step of providing a substrate for producing cell aggregates which is provided with a plurality of spots comprising a copolymer which contains a recurring unit derived from a monomer represented by the following formula (I): 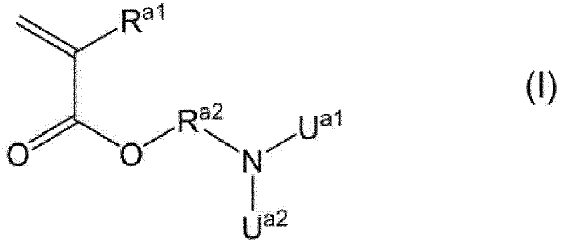 [wherein
   U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms], and a recurring unit derived from a monomer represented by the following formula (II): 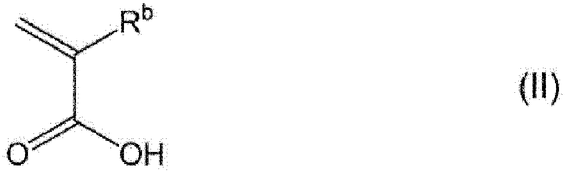 [wherein
   R^{b} represents a hydrogen atom or a linear or branched alkyl group having 1 to 5 carbon atoms] on a substrate having an ability to suppress adhesion of cells; a step of seeding human cartilage progenitor cells which are positive for PRRX1 protein and derived from pluripotent stem cells on the substrate; a step of producing cell aggregates by culturing the cells; and a step of culturing the aggregates to produce a cartilage tissue.
[2] The producing method described in the above-mentioned [1], wherein a molar ratio of the recurring unit derived from the monomer represented by the formula (I) based on a sum of the above-mentioned recurring unit derived from the monomer represented by the formula (I) and the above-mentioned recurring unit derived from the monomer represented by the formula (II) is 99 mol% to 51 mol%.
[3] The producing method described in the above-mentioned [1] or [2], wherein the above-mentioned copolymer further contains a structural unit derived from a monomer represented by the following formula (III): 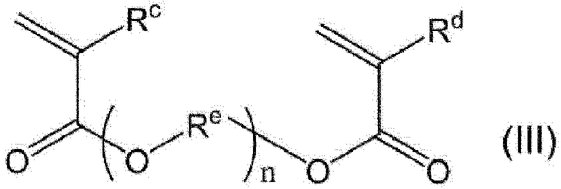 [wherein
   R^{c} and R^{d} each independently represent a hydrogen atom or a linear or branched alkyl group having 1 to 5 carbon atoms, R^{e} represents a linear or branched alkylene group having 1 to 5 carbon atoms and n represents a number of 1 to 50].
[4] The producing method described in any of the above-mentioned [1] to [3], wherein the above-mentioned substrate having an ability to suppress adhesion of cells has a coating film containing a copolymer (P) which contains a recurring unit containing a group represented by the following formula (a) and a recurring unit containing a group represented by the following formula (b): 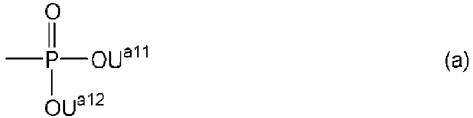 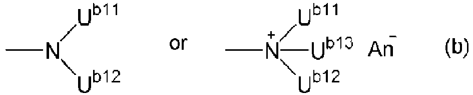 [wherein
   U^{a11}, U^{a12}, U^{b11}, U^{b12} and U^{b13} each independently represent a hydrogen atom or a linear or branched alkyl group having 1 to 5 carbon atoms, An⁻ represents an anion selected from the group consisting of a halide ion, an inorganic acid ion, a hydroxide ion and an isothiocyanate ion]
   on at least a part of the surface thereof.
[5] The producing method described in any of the above-mentioned [1] to [4], wherein the culture in the step of producing the cartilage tissue is carried out in a culture medium containing agar.
[6] The producing method described in the above-mentioned [5], wherein a weight average molecular weight of the agar is 10,000 to 60,000, and a content of the agar is 0.005 (w/v) % or more and less than 2 (w/v) % based on the whole amount of the culture medium.

### EFFECTS OF THE INVENTION

The production method of the present invention is advantageous for the safe use of cartilage tissue for regenerative medicine since it uses quality-controlled cartilage progenitor cells as a raw material and the cells are treated to induce chondrogenic differentiation to obtain cartilage tissue, and since other cells than the cartilage cells are not contained in the produced cartilage tissue. Further, it is advantageous in terms of cost by carrying out production of cartilage tissue from cartilage progenitor cells under the prescribed culture conditions, it is possible to produce large cartilage tissue pieces from a small number of cells as well as efficient cell growth.

### BRIEF DESCRIPTION OF THE DRAWINGS

Fig. 1 are whole image (upper left) and magnified image (lower left) of the cell aggregates formed on the substrate for producing cell aggregates obtained in Example 1, and whole image (upper right) and magnified image (lower right) of the cell aggregates visualized by expression of the red fluorescent protein (tdTomato).
Fig. 2 is a photograph of a stereoscopic microscope of a cartilage tissue obtained in Example 1.
Fig. 3 is an inverted micrograph (bright field) of the cartilage tissue obtained in Example 1.
Fig. 4 are images of tissue sections of the cartilage tissue obtained in Example 1, which are subjected to hematoxylin (HE) stain (left), alcian blue stain (center), or safranin O stain (right).
Fig. 5 are, in Evaluation Example 1, images in which tissue sections collected 4 weeks after transplantation of the cartilage tissue obtained in Example 1, which are subjected to HE stain (left), safranin O stain (center), or toluidine blue stain (right).

### EMBODIMENTS TO CARRY OUT THE INVENTION

### <Method for producing cartilage tissue >

The method for producing a cartilage tissue of the present invention comprises
(1) a step of providing a substrate for producing cell aggregates which is provided with a plurality of spots comprising a copolymer containing recurring units derived from monomers represented by the formulae (I) and (II) on a substrate having an ability to suppress adhesion of cells;
(2) a step of seeding human cartridge progenitor cells which are positive for PRRX1 protein and derived from pluripotent stem cells on the substrate;
(3) a step of producing cell aggregates by culturing the cells; and
(4) a step of culturing the aggregates to produce a cartilage tissue.

### Step (1)

In Step (1), a substrate for producing cell aggregates is provided. The substrate for producing cell aggregates according to the present invention is provided with a plurality of spots comprising a copolymer containing recurring units derived from monomers represented by the formulae (I) and (II) on the substrate having an ability to suppress adhesion of cells.

### (Copolymer)

The above-mentioned copolymer contains a recurring unit derived from a monomer represented by the following formula (I): 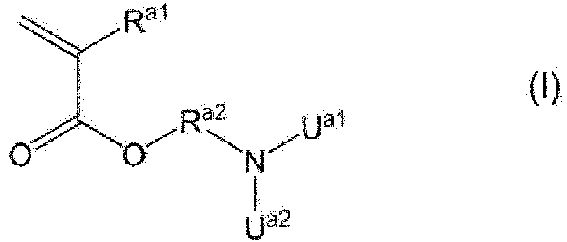 [wherein
U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms], and a recurring unit derived from a monomer represented by the following formula (II): 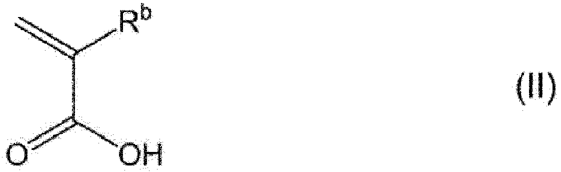 [wherein
R^{b} represents a hydrogen atom or a linear or branched alkyl group having 1 to 5 carbon atoms].

In the present specification, unless otherwise specifically defined, as the "linear or branched alkyl group having 1 to 5 carbon atoms", there may be mentioned, for example, a methyl group, an ethyl group, an n-propyl group, an isopropyl group, an n-butyl group, an isobutyl group, an s-butyl group, a t-butyl group, an n-pentyl group, a 1-methylbutyl group, a 2-methylbutyl group, a 3-methylbutyl group, a 1,1-dimethylpropyl group, a 1,2-dimethylpropyl group, a 2,2-dimethylpropyl group or a 1-ethylpropyl group.

R^{a1} and R^{b} are preferably each independently selected from a hydrogen atom and a methyl group.

U^{a1} and U^{a2} are preferably each independently selected from a hydrogen atom, a methyl group, an ethyl group, an n-propyl group, an isopropyl group and an n-butyl group, preferably a methyl group or an ethyl group, and most preferably a methyl group.

In the present specification, unless otherwise specifically defined, as the "linear or branched alkylene group having 1 to 5 carbon atoms", there may be mentioned, for example, a methylene group, an ethylene group, a propylene group, a trimethylene group, a tetramethylene group, a 1-methylpropylene group, a 2-methylpropylene group, a dimethylethylene group, an ethylethylene group, a pentamethylene group, a 1-methyl-tetramethylene group, a 2-methyl-tetramethylene group, a 1,1-dimethyl-trimethylene group, a 1,2-dimethyl-trimethylene group, a 2,2-dimethyl-trimethylene group, a 1-ethyl-trimethylene group, etc. Among these, as R^{a2}, it is preferably selected from an ethylene group and a propylene group.

Accordingly, as the monomer represented by the above-mentioned formula (I), there may be mentioned 2-N,N-dimethylaminoethyl methacrylate, N,N-dimethylaminomethyl methacrylate, etc., and preferably 2-N,N-dimethylaminoethyl methacrylate. As the monomer represented by the above-mentioned formula (II), there may be mentioned acrylic acid, methacrylic acid, etc., and preferably methacrylic acid.

A molar ratio of the recurring unit derived from the monomer represented by the formula (I) in the above-mentioned copolymer to the sum of the recurring unit derived from the monomer represented by the formula (I) and the recurring unit derived from the monomer represented by the formula (II) is preferably 99 mol% to 51 mol%, more preferably 98 mol% to 60 mol%, further preferably 95 mol% to 70 mol%, and further more preferably 93 mol% to 70 mol%. If the molar ratio of the recurring unit derived from the monomer represented by the formula (II) is 50 mol% or more, the anionic property of the copolymer becomes excessive and adhesive force of the cells tends to be lowered. Incidentally, in the present specification, unless otherwise specified, the molar ratio of the recurring unit derived from each monomer can be replaced with the ratio calculated from the charged amount (molar amount) of the monomer.

The above-mentioned copolymer may further contain, together with the recurring unit derived from the monomer represented by the formula (I)/the formula (II), a structural unit derived from a monomer having two or more carbon-carbon unsaturated bonds. The monomer having two or more carbon-carbon unsaturated bonds is specifically a monomer having two or more carbon-carbon double bonds and, for example, there may be mentioned a polyfunctional acrylate compound, a polyfunctional acrylamide compound, a polyfunctional polyester, or an isoprene compound, etc.

Preferred specific examples may be mentioned monomers represented by the following formulae (III) to (V).

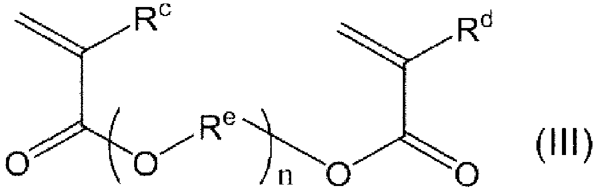

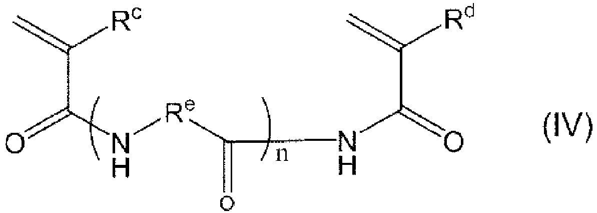

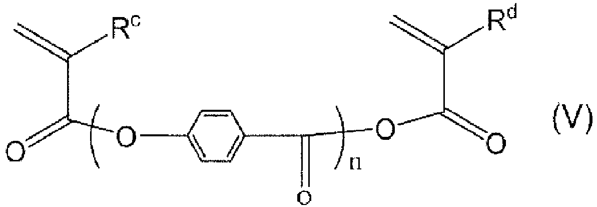

In the formulae, R^{c} and R^{d} each independently represent a hydrogen atom or a linear or branched alkyl group having 1 to 5 carbon atoms, R^{e} represents a linear or branched alkylene group having 1 to 5 carbon atoms and n represents a number of 1 to 50. Among these, the monomer represented by the formula (III) is preferable.

It is preferable that R^{c} and R^{d} are each independently selected from a hydrogen atom and a methyl group.

R^{e} is preferable selected from a methylene group, an ethylene group and a propylene group, and an ethylene group is more preferable.
n is a number of 1 to 50, n is preferably a number of 1 to 30, and n is more preferably a number of 1 to 10.

A molar ratio of the recurring unit derived from the monomer having two or more carbon-carbon unsaturated bonds (typically, the monomer represented by the formulae (III) to (V)) in the above-mentioned copolymer is preferably 0 mol% to 5 mol%, and more preferably 0 mol% to 3 mol%. If the molar ratio of the recurring unit derived from the monomer having two or more carbon-carbon unsaturated bonds exceeds 5 mol%, there is a fear of making difficult to produce due to gelation during production by becoming higher molecular weight due to excessive crosslinking.

The above-mentioned copolymer may further contain, as an optional addition monomer component(s), a recurring unit(s) derived from an ethylenically unsaturated monomer, or a polysaccharide or a derivative thereof. Examples of the ethylenically unsaturated monomer may be mentioned one kind or two or more kinds of ethylenically unsaturated monomers selected from the group consisting of a (meth)acrylic acid ester; vinyl acetate; vinylpyrrolidone; ethylene; vinyl alcohol; and a hydrophilic functional derivative thereof. Examples of the polysaccharide or a derivative thereof may be mentioned a cellulose-based polymer such as hydroxyalkyl cellulose (for example, hydroxyethyl cellulose or hydroxypropyl cellulose), etc., starch, dextran and curdlan.

The hydrophilic functional derivative refers to an ethylenically unsaturated monomer having a hydrophilic functional group or structure. Examples of the hydrophilic functional group or structure may be mentioned a betaine structure; an amide structure; an alkylene glycol residue; an amino group; and a sulfinyl group, etc.

The betaine structure means a monovalent or a divalent group of a compound having an amphoteric center of a quaternary ammonium type cation structure and an acidic anion structure and may be mentioned, for example, a phosphorylcholine group: 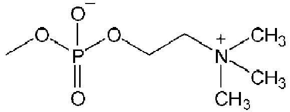 Examples of the ethylenically unsaturated monomer having such a structure may be mentioned 2-methacryloyloxyethyl phosphorylcholine (MPC), etc.

The amide structure means a group represented by the following formula: 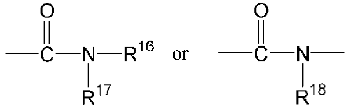 [here, R¹⁶, R¹⁷ and R¹⁸ are each independently a hydrogen atom or an organic group (for example, a linear or branched alkyl group having 1 to 5 carbon atoms which may be substituted, etc., specifically, a methyl group, an isopropyl group, a hydroxymethyl group or a hydroxyethyl group, etc.)].
Examples of the ethylenically unsaturated monomer having such a structure may be mentioned (meth)acrylamide, N-isopropylacrylamide, N-(hydroxymethyl) (methacrylamide, etc. Further, the monomer or the polymer having such a structure is disclosed in, for example, JP 2010-169604A, etc.

The alkylene glycol residue means an alkyleneoxy group (-Alk-O-) which remains after a hydroxyl group(s) at one side terminal or both terminals of the alkylene glycol (HO-Alk-OH; here, Alk is an alkylene group having 1 to 10 carbon atoms) is/are subjected to condensation reaction with other compound(s), and a poly(alkyleneoxy) group in which alkyleneoxy units are repeated is also included. Examples of the ethylenically unsaturated monomer having such a structure may be mentioned 2-hydroxyethyl (meth)acrylate, methoxypolyethylene glycol (meth)acrylate, etc.
Further, the monomer having such a structure is disclosed in, for example, JP 2008-533489A, etc.

The amino group means a group represented by the formula: -NH₂, -NHR¹⁹ or -NR²⁰R²¹ [here, R¹⁹, R²⁰ and R²¹ are each independently an organic group (for example, a linear or branched alkyl group having 1 to 5 carbon atoms, etc.)]. In the amino group in the present invention, a quaternized or chlorinated amino group is included. Examples of the ethylenically unsaturated monomer having such a structure may be mentioned dimethylaminoethyl (meth)acrylate, 2-(t-butylamino)ethyl (meth)acrylate, methacryloylcholine chloride, etc.

The sulfinyl group means a group represented by the following formula: 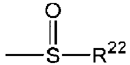 [here, R²² is an organic group (for example, an organic group having 1 to 10 carbon atoms, and preferably an alkyl group having 1 to 10 carbon atoms and having one or more hydroxyl groups, etc.)].
As a method for introducing the sulfinyl group, there may be mentioned the method disclosed in the publication of JP 2014-48278A, etc.

The copolymer of the present application can be produced by the method known *per se* (for example, the methods described in JP 2014-162865A and WO 2020/040247).

A number average molecular weight (Mn) of the above-mentioned copolymer is 20,000 to 1,000,000, and further preferably 50,000 to 800,000.

A ratio (Mw/Mn) of the weight average molecular weight (Mw) and the above-mentioned number average molecular weight (Mn) of the above-mentioned copolymer is 1.01 to 10.00, preferably 1.2 to 8.0, preferably 1.4 to 6.0, preferably 1.5 to 5.0, and preferably 1.6 to 4.5.

The above-mentioned number average molecular weight (Mn) and the number average molecular weight (Mn) can be obtained by, for example, Gel Filtration Chromatography.

It is possible to form cell aggregates (spheroids) by peeling them after adhering cells, by utilizing the above-mentioned copolymer. Incidentally, the cell aggregates refers to a structure formed as a result of aggregating cells, and its shape is not limited to a spherical shape or a ring shape, etc.

### (Substrate)

The substrate for producing cell aggregates used in the present invention can be produced by coating the above-mentioned copolymer onto the surface of the substrate and drying the same. Here, the "surface" refers to a surface in contact with the contents such as cells or cell culture medium, etc., and in particular, when the substrate is a part of the cell culture container, it refers to the bottom surface among the surfaces in contact with the contents.

A shape of the surface of the substrate may be plane surface or may have unevenness, and a plane shape is preferable. Also, the substrate may be a so-called cell culture container or a part thereof. As the cell culture container, there may be mentioned, for example, petri dishes or dishes such as petri dishes generally used for culture of cells, dishes for tissue culture, multi dishes, etc., flasks such as cell culture flasks, spinner flasks, etc., bags such as plastic bags, Teflon (Registered Trademark) bags, culture bags, etc., plates such as microplates, microwell plates, multi plates, multiwell plates, etc., chamber slides, tubes, trays, and a bottle such as roller bottles, etc.

The material of the substrate may be mentioned, for example, glass, a metal, a metal containing compound or a semi-metal containing compound, activated charcoal or a resin. The metal may be mentioned a typical metal: an aluminum group element: Al, Ga, In; an iron group element: Fe, Co, Ni; a chromium group element: Cr, Mo, W, U; a manganese group element: Mn, Re; a noble metal: Cu, Ag, Au, etc. The metal containing compound or the semi-metal containing compound may be mentioned, for example, ceramics comprising a metal oxide as a basic component, which are a sintered body baked by a heat treatment at a high temperature, a semiconductor such as silicon, an inorganic solid material including a molded product of an inorganic compound such as a metal oxide or a semi-metal oxide (silicon oxide, alumina, etc.), a metal carbide or a semi-metal carbide, a metal nitride or a semi-metal nitride (silicon nitride, etc.), a metal boride or a semi-metal boride, etc., aluminum, nickel-titanium and stainless (SUS304, SUS316, SUS316L, etc.).

As the resin, it may be either of a natural resin or a derivative thereof, or a synthetic resin, as the natural resin, there may be mentioned, for example, cellulose, cellulose triacetate (CTA), nitrocellulose (NC), cellulose to which dextran sulfate has been fixed, etc., and as the synthetic resin, there may be preferably used polyacrylonitrile (PAN), polyimide (PI), polyester-based polymer alloy (PEPA), polystyrene (PS), polysulfone (PSF), polyethylene terephthalate (PET), polymethyl methacrylate (PMMA), polyvinyl alcohol (PVA), polyurethane (PU), ethylene vinyl alcohol (EVAL), polyethylene (PE), polyester, polypropylene (PP), polyvinylidene fluoride (PVDF), polyether sulfone (PES), polycarbonate (PC), cycloolefin polymer (COP), polyvinyl chloride (PVC), polytetrafluoroethylene (PTFE), ultra-high molecular weight polyethylene (UHPE), polydimethylsiloxane (PDMS), acrylonitrile-butadienestyrene resin (ABS) or Teflon (Registered Trademark).

In the production of the substrate for producing cell aggregates used in the present invention, at the time of coating the above-mentioned copolymer on the surface of the substrate in a spot state and drying the same, it is not necessary to treat it at a high temperature, so that a resin having low heat resistance, etc., can be also applied. A material(s) of the substrate may be one kind or a combination of two or more kinds.

### (Spot)

The substrate for producing cell aggregates used in the present invention is provided with a plurality of spots comprising the above-mentioned copolymer. A ratio of the total area of the spots to the surface area of the substrate is not particularly limited, and for example, it is 30 % or more, the diameter of each spot is, for example, 50 to 5,000 µm, and he distance between the spots is to be, for example, 30 to 1,000 µm. A shape of the spots is not particularly limited, and for example, it is substantially circular shape or a quadrangular shape, and a spot of substantially circular shape is preferable.

The ratio of the total area of the spots to the surface area of the substrate, the diameter of each spot and the distance between the spots may be appropriately selected from a predetermined range according to the kind of cells and the substrate to be used, the desired size of the cell aggregate, etc., and the ratio of the total area of the spots to the substrate is preferably 30 % or more, 40 % or more, and 50 % or more, and preferably 99% or less, the diameter of each spot is 50 to 5,000 µm, and preferably 300 to 3,000 µm, and the distance between the spots is 30 to 1,000 µm, and preferably 100 to 500 µm.

By arranging independent micro-sized regions (spots) to which cells can adhere on a substrate having an ability to suppress adhesion of cells, in such a high density and preferably regularly, a plurality of spheroids of uniform size can be formed at one time on one substrate (container). There is a merit in the point of adjusting a size of the cell aggregates according to the regulation of the adhesion area (cell aggregates having an optional size can be produced), etc., as compared with the cell aggregates produced on a conventional cell low-adhesion plate by non-adhesive culture.

The spot comprising the above-mentioned copolymer can be formed by coating a base agent containing a copolymer, and preferably the above-mentioned copolymer. The base agent can be prepared by mixing the above-mentioned copolymer with a water-containing solution by a method known *per se.* Such a base agent is useful for promoting formation of the cell aggregates.

The water-containing solution may be mentioned water, a salt-containing aqueous solution such as a physiological saline or a phosphate buffer solution, etc., or a mixed solvent in which water or the salt-containing aqueous solution and an alcohol are combined. As the alcohol, there may be mentioned an alcohol having 2 to 6 carbon atoms, for example, ethanol, propanol, isopropanol, 1-butanol, 2-butanol, isobutanol, t-butanol, 1-pentanol, 2-pentanol, 3-pentanol, 1-heptanol, 2-heptanol, 2,2-dimethyl-1-propanol (=neopentyl alcohol), 2-methyl-1-propanol, 2-methyl-1-butanol, 2-methyl-2-butanol (=t-amyl alcohol), 3-methyl-1-butanol, 3-methyl-3-pentanol, cyclopentanol, 1-hexanol, 2-hexanol, 3-hexanol, 2,3-dimethyl-2-butanol, 3,3-dimethyl-1-butanol, 3,3-dimethyl-2-butanol, 2-ethyl-1-butanol, 2-methyl-1-pentanol, 2-methyl-2-pentanol, 2-methyl-3-pentanol, 3-methyl-1-pentanol, 3-methyl-2-pentanol, 3-methyl-3-pentanol, 4-methyl-1-pentanol, 4-methyl-2-pentanol, 4-methyl-3-pentanol and cyclohexanol, and these may be used alone or may be used a mixed solvent in combination thereof.

Further, to the base agent may be added, in addition to the above-mentioned copolymer and the solvent, other substance(s) within the range which do not impair the properties of the resulting base film, if necessary. As the other substance(s), there may be mentioned pH adjusting agents, crosslinking agents, preservatives, surfactants, primers that enhance adhesiveness to the container or the substrate, fungicides and saccharides, etc.

As the system of coating the base agent, there may be employed, for example, an inkjet method, a screen printing method, a slit coating method, a roll-to-roll method, etc., and preferably carried out by a printing technique of the inkjet method or the screen printing method, etc.

As another coating method, there may be employed, for example, a method in which a substrate in which the non-formed portion of the spot is protected is immersed in the above-mentioned base agent as the case may be, a base agent is added to the substrate (container) in which the non-formed portion of the spot is protected as the case may be and allowed to stand for a predetermined time, etc., and in the case of a substrate, or a cell culture container as an embodiment, it is carried out by the method in which the base agent is added to a container the non-formed portion of the spot is protected as the case may be and allowed to stand for a predetermined time. Addition can be carried out by adding, for example, 0.5 to 1-fold amount of the base agent based on the whole volume of the container using a syringe, etc. Allowing to stand can be practiced by optionally selecting a time and temperature depending on the material of the container or the substrate, the kind of the base agent for cell culture, and is practiced, for example, 1 minute to 24 hours, preferably 5 minutes to 3 hours at 10 to 80°C. According to the procedure, the substrate for producing cell aggregates can be produced.

Also, the spots on the surface of the substrate obtained by such a method can be used as a substrate for producing cell aggregates without subjecting to the drying step as such, or after washing using water or a culture medium of the sample applied to cell culture (for example, water, buffer solution, culture medium, etc.).

That is, after forming the spots on the surface of the substrate, within 48 hours, preferably within 24 hours, further preferably within 12 hours, further preferably within 6 hours, further preferably within 3 hours, further preferably within 1 hour, without subjecting to a drying step as such, or after washing using water or a culture medium of a sample applied to cell culture (for example, water, buffer solution, culture medium, etc., particularly preferably culture medium (for example, DMEM medium (Dulbecco's modified eagle medium)), it can be used as a substrate for producing cell aggregates.

The substrate for producing cell aggregates may be subjected to a drying step. The drying step is carried out under atmosphere or under vacuum, preferably at a temperature in the range of -200°C to 200°C. According to the drying step, it completely adheres to the substrate by removing the solvent in the above-mentioned base agent.

The spots can be also formed, for example, by drying at room temperature (10°C to 35°C, preferably 20°C to 30°C, for example, 25°C), but in order to form the spots more quickly, for example, it may be dried at 40°C to 50°C. If the drying temperature is lower than -200°C, a refrigerant which is not common must be used so that it is not versatile, and it takes a long time to dry due to solvent sublimation so that efficiency is bad. If the drying temperature exceeds 200°C, thermal decomposition of the copolymer occurs. A more preferable drying temperature is 10°C to 180°C, and a more preferable drying temperature is 20°C to 150°C.

The substrate for producing cell aggregates used in the present application is produced through the above simple and easy steps.

Also, in order to remove impurities, unreacted monomers, etc., remained in the spots (coating film), a step of washing with at least one kind of a solvent selected from water and an aqueous solution containing an electrolyte(s) may be carried out. Washing is desirably carried out with running water washing or ultrasonic washing, etc. The above-mentioned water and aqueous solution containing an electrolyte(s) may be a material heated in the range of, for example, 40°C to 95°C. The aqueous solution containing an electrolyte(s) is/are preferably PBS, physiological saline (containing only sodium chloride), Dulbecco's phosphate buffered physiological saline, Tris buffered physiological saline, HEPES buffered physiological saline and Veronal buffered physiological saline, and particularly preferably PBS. After fixing, the coating film does not dissolve out and remains firmly fixed to the substrate even when it is washed with water, PBS and an alcohol, etc.

Regarding the film thickness of the spot (coating film), the maximum film thickness and the minimum film thickness are in the range of 1 to 1,000 nm, and preferably in the range of 5 to 500 nm.

The substrate for producing cell aggregates to be used in the present invention is produced by a substrate having an ability to suppress adhesion of cells. As such a substrate, a commercially available cell culture dish having undergone cell low-adhesion treatment, a cell incubator having an ability to suppress adhesion of cells, etc., may be used, and for example, a cell culture container described in JP 2008-61609A can be used, but is not limited to this. Or else, the substrate may be subjected to a treatment of suppressing adhesion of cells before formation of the above-mentioned spots (coating film). The substrate having an ability to suppress adhesion of cells can be produced, for example, through a step of coating a known composition for forming a coating film having an ability to suppress adhesion of cells. As the composition for forming a coating film having an ability to suppress adhesion of cells, it is preferable to contain steps of coating a composition for forming a coating film which contains a copolymer (P) which contains a recurring unit having an organic group represented by the following formula (a) and a recurring unit having an organic group represented by the following formula (b): 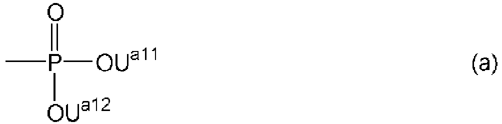 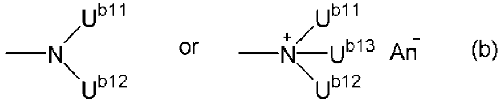 [wherein
U^{a11}, U^{a12}, U^{b11}, U^{b12} and U^{b13} each independently represent a hydrogen atom or a linear or branched alkyl group having 1 to 5 carbon atoms, An⁻ represents an anion selected from the group consisting of a halide ion, an inorganic acid ion, a hydroxide ion and an isothiocyanate ion]
and a solvent on the surface of a container or a substrate and drying. The above-mentioned coating film may be contained at least a part of the surface of the substrate, and it is preferable that it is coated over the entire surface for producing cell aggregates (that is, the surface where the spots of the present application are present), or over the entire surface of the substrate.

The linear or branched alkyl group having 1 to 5 carbon atoms is the same as mentioned above.

As the above-mentioned composition for forming a coating film, for example, a composition for forming a coating film described in WO 2014/196650 can be used.

The coating method of the above-mentioned composition for forming a coating film is not particularly limited, and a usual coating method such as spin coating, dip coating, solvent casting method, etc., is used.

The drying step of the above-mentioned coating film is carried out under atmosphere or under vacuum at a temperature in the range of -200°C to 180°C. According to the drying step, the solvent in the above-mentioned composition for forming a coating film is removed, and the formula (a) and the formula (b) in the above-mentioned copolymer form an ionic bond and are completely adhered to the substrate.

The above-mentioned coating film can be also formed, for example, by drying at room temperature (10°C to 35°C, for example, 25°C), but in order to form the coating film more quickly, it may be dried at, for example, 40°C to 50°C. In addition, a drying step at an extremely low temperature to a low temperature (around -200°C to -30°C) by a freeze-drying method may be used. Freeze-drying is called to as vacuum freeze-drying, which is a method of usually cooling what is desired to be dried with a refrigerant and removing the solvent by sublimation in a vacuum state. A general refrigerant used in freeze-drying may be mentioned a mixed culture medium of dry ice and methanol (-78°C), liquid nitrogen (-196°C), etc.

If the drying temperature is -200°C or lower, a refrigerant which is not common must be used so that it is not versatile, and it takes a long time to dry due to solvent sublimation so that efficiency is bad. If the drying temperature is 200°C or higher, the ionic bonding reaction on the surface of the coating film proceeds excessively and the surface loses its hydrophilicity whereby the ability to suppress adhesion of biological substances is not exhibited. A more preferable drying temperature is 10°C to 180°C, and a more preferable drying temperature is 25°C to 150°C.

After drying, it is desirable to carry out washing such as running water washing or ultrasonic washing, etc., with one or more solvents selected from water and an aqueous solution containing an electrolyte(s) in order to eliminate impurities, unreacted monomers, etc., remained on the above-mentioned coating film, and further to adjust the ion balance of the copolymer in the film. The above-mentioned water and aqueous solution containing an electrolyte(s) may be a material heated in the range of, for example, 40°C to 95°C. The aqueous solution containing an electrolyte(s) is preferably PBS, physiological saline (containing only sodium chloride), Dulbecco's phosphate buffered physiological saline, Tris buffered physiological saline, HEPES buffered physiological saline and Veronal buffered physiological saline, and particularly preferably PBS. After fixing, the coating film does not dissolve out and remains firmly fixed to the substrate even when it is washed with water, PBS and an alcohol, etc. Even when a biological substance is attached to the formed coating film, it can be easily removed by washing with water, etc., and the surface of the substrate on which the above-mentioned coating film is formed has an ability to suppress adhesion of the biological substance.

A film thickness of the above-mentioned coating film is preferably 5 to 1,000 nm, and further preferably 5 to 500 nm.

Having an ability to suppress cell adhesion means that, for example, a relative absorbance (WST O.D. 450 nm) (%) ((absorbance (WST O.D.450 nm) of Example)/(absorbance (WST O.D. 450 nm) of Comparative Example)) when compared with no coating film or without cell low-adhesion treatment by the fluorescence microscope carried out by the method described in, for example, Example of WO2016/093293 is 50% or less, preferably 30% or less, and further preferably 20% or less.

### Step (2)

In Step (2), human cartilage progenitor cells which are positive for PRRX1 protein derived from pluripotent stem cells are seeded onto the above-mentioned substrate for producing cell aggregates.

The terms "pluripotent stem cell" mean a cell that has both an ability to self-renew and an ability to differentiate into other cells of multiple lines (pluripotency), and the terms "progenitor cell" mean a cell that is in the process of differentiating from a stem cell into a functional cell which is a final differentiation destination.
Accordingly, the human cartilage progenitor cells derived from pluripotent stem cells mean cells that have been induced to differentiate from the pluripotent stem cell as raw material cells and are in the process of being differentiated into chondrocytes that are the final differentiation destination. Here, as pluripotent stem cells, there may be mentioned, for example, pluripotent cells (cells that have an ability to differentiate into all somatic cells and germ line cells) such as embryonic stem cells (ES cells), induced pluripotent stem cells (iPS cells), etc.

Further, in the present invention, "human cartilage progenitor cells" are positive for PRRX1 protein. PRRX1 (Paired related homeobox 1) protein is one of the transcription factors having a homeodomain, and is known to be specifically expressed in limb buds derived from lateral plate mesoderm and head mesoderm derived from paraxial mesoderm during development.

The nucleotide sequence of cDNA of PRRX1 gene and the amino acid sequence of the PRRX1 protein of human (Homo sapiens) are registered in GenBank provided by US bionics information center (NCBI; National Center for Biotechnology Information) with the following accession numbers. Incidentally, when a plurality of revisions (revision) are registered, it is understood to refer to the latest revision.
- Human PRRX1 gene: NM_006902 (NM_006902.5), NM_022716 (NM_022716.4)
- Human PRRX1 protein: NP_008833 (NP_008833.1), NP_073207 (NP_073207. 1)

The fact that cells are positive for PRRX1 protein can be detected by a known method. For example, there may be mentioned detection by a reporter gene whose expression is regulated by a transcription promoter sequence of the PRRX1 gene, detection by immunostaining using an antibody specific for the PRRX1 protein, etc.

Cartilage progenitor cells which are positive for PRRX1 protein can be produced, for example, by a method including the following steps:
- a step of inducing differentiation of lateral plate mesoderm cells from pluripotent stem cells,
- a step of culturing cells induced to differentiate by the above-mentioned step in an environment that activates Wnt signal to prepare limb bud mesenchymal cells,
- a step of culturing limb bud mesenchymal cells prepared by the above-mentioned step in an environment that activates Wnt signal to prepare cartilage progenitor cells.

In the above-mentioned method, as the pluripotent stem cells to be used as raw material cells, for example, embryonic stem cell (ES cells), induced pluripotent stem cells (iPS cells), etc., can be used. As ES cells and iPS cells, newly prepared ones or already established ones can be used.

In the above-mentioned method, first, lateral plate mesoderm cells are induced to differentiate from pluripotent stem cell. As a means of inducing differentiation of lateral plate mesoderm cells from pluripotent stem cells, known means can be used. For example, a method according to the method described in the literature: Loh et al, 2016, Cell, 451-467 can be employed. Specifically, the pluripotent stem cells are first induced to differentiate into primitive streak (Mid-primitive streak), and then the primitive streak is induced to differentiate into the lateral plate mesoderm cells.

The induction of differentiation of the lateral plate mesoderm cells can be confirmed, for example, by detecting the expression of the HAND1 protein, which is a specific marker of the lateral plate mesoderm cells.

Then, the differentiation-induced lateral plate mesoderm cells are cultured in an environment that activates Wnt signal, to induce differentiation of lateral plate mesoderm-derived PRRX1 positive cells which is positive for PRRX1 protein. Incidentally, in order to reduce the influence of the previous culture environment, it is preferable to carry out culture in an environment that activates Wnt signal after appropriately washing with a PBS buffer solution, etc.

Induction of differentiation from lateral plate mesoderm cells to limb bud mesenchymal cells is also preferably carried out in the absence of an FGF signal activator such as FGF2, etc., and more preferably carried out in an environment that activates Wnt signal and in the presence of one kind, two kinds or three kinds selected from the group consisting of TGFβ signal suppressors, BMP signal suppressors and Hedgehog signal suppressors, and further preferably carried out in the presence of TGFβ signal suppressors, BMP signal suppressors, TGFβ signal suppressors, and Hedgehog signal suppressors.

Culture in an environment that activates Wnt signal can be carried out, for example, by culturing in the presence an effective amount of a Wnt signal activator. The Wnt signal activator is a material that enhances signal transduction (in particular, the canonical Wnt pathway) mediated by Wnt. As the Wnt signal activator, there may be mentioned GSK3β inhibitors, Wnt family proteins, etc. For example, as the GSK3β inhibitors, there may be mentioned CHIR99021 (6-[[2-[[4-(2,4-Dichlorophenyl)-5-(5-methyl-1H-imidazol-2-yl)-2-pyrimidinyl]amino]ethyl]amino]-3-pyridinecarbonitrile), XAV939 (3,5,7,8-Tetrahydro-2-[4-(trifluoromethyl)phenyl]-4H-thiopyrano[4,3-d]pyrimidin-4-one), LiC1, etc. When CHIR99021 is used as the Wnt signal activator, an amount thereof to be added may be made, for example, about 0.1 to 20 µM, and preferably 1 to 10 µM.

The BMP signal suppressor is a material that is to suppress (inhibit) signal transduction mediated by BMP. As the BMP signal suppressor, there may be mentioned LDN193189 (4-[6-[4-(1-Piperazinyl)phenyl]pyrazolo[1,5-a]pyrimidin-3-yl]quinoline) or its salt (for example, hydrochloride), DMH-1, etc., and an amount thereof to be added may be made, for example, about 0.1 to 10 µM, and preferably 0.2 to 5 µM.

The TGFβ signal suppressor is a material that is to suppress (inhibit) signal transduction mediated by TGFβ. As the TGFβ signal activator, there may be mentioned A-83-01 (3-(6-methyl-2-pyridinyl)-N-phenyl-4-(4-quinolynyl)-1H-pyrazol-1-carbothioamide), SB431542, etc., and an amount thereof to be added may be made, for example, about 0.1 to 10 µM, and preferably 0.2 to 5 µM.

Hedgehog signal suppressor is a material that is to suppress (inhibit) signal transduction mediated by Hedgehog. As the Hedgehog signal activator, there may be mentioned vismodegib, cyclopamine, sonidegib, etc., and an amount thereof to be added may be made, for example, about 10 nM to 1 µM, and preferably about 50 nM to 500 nM.

As culture, there may be exemplified by a method in which culture is carried out under the conditions of about 37°C or so and a carbon dioxide concentration of about 5% or so, but it is not limited to this. Culture under the above-mentioned conditions can be carried out, for example, using a known CO₂ incubator while controlling the temperature and the CO₂ concentration.

Culture can be carried out in two-dimensional cell culture (planar culture). Two-dimensional cell culture can be carried out by subjecting to coating treatment of the culture apparatus to promote adhesion of cells, if necessary.

The term of performing culture in an environment that activates Wnt signal is not particularly limited, and can be made, for example, 6 hours to 4 days or so, preferably 1 day to 3 days or so, more preferably 2 days or so (48 hours or so). If necessary, exchange of the culture medium can be carried out. The culture conditions are preferably in accordance with the usual method.

In culture, passage may be carried out, if necessary. When passage is carried out, the cells are recovered before or immediately after reaching confluent state and seeded into a new culture medium. In addition, the culture medium can be appropriately exchanged.

As the culture medium, it is preferable to use serum-free medium such as IMDM medium, F12 medium, or a mixed medium thereof, etc. In the state of not containing a component(s) derived from animal(s), differentiation induction and maintenance culture are possible. In addition, to the above-mentioned serum-free medium may be added various kinds of medium additives including antimicrobial agents such as streptomycin, penicillin, etc., non-essential amino acids (NEAA), ROCK inhibitor (for example, Y-27632 ((R)-(+)-trans-N-(4-Pyridyl)-4-(1-aminoethyl)-cyclohexanecarboxamide-2HCl)), hormones such as insulin, proteins such as transferrin, albumin, etc., lipids, polyvinyl alcohol, monothioglycerol, etc.

Then, the prepared limb bud mesenchymal stem cells are cultured in an environment that activates Wnt signal to induce differentiation into cartilage progenitor cells which are positive for PRRX1 protein. Incidentally, the environment that activates Wnt signal have the same meanings as mentioned above. In addition, it is preferable that the induction of differentiation from limb bud mesenchymal cells to cartilage progenitor cells is carried out in the presence of an FGF signal activator such as FGF2, etc.

In this way, cartilage progenitor cells which are positive for PRRX1 protein are produced. Specific embodiments are as described in Example mentioned later. Also, the description of WO 2021/054449 can be referred to. In addition, the cartilage progenitor cells thus obtained can be subjected to expanded culture in the same lot, and can be stocked under certain quality control conditions.

Seeding of human cartilage progenitor cells onto the above-mentioned substrate for producing cell aggregates is carried out by a method known *per se,* in which the cells are suspended in a culture medium, for example, the culture medium used in culture in the subsequent Step (3) to prepare a cell suspension, and this is added to the above-mentioned substrate for producing cell aggregates, etc.

### Step (3)

In Step (3), human cartilage progenitor cells which are positive for PRRX1 protein and derived from pluripotent stem cells are cultured to produce cell aggregates.

Culture is not particularly limited as long as it is a method capable of producing cell aggregates from the above-mentioned human cartilage progenitor cells, and is appropriately selected depending on the properties of the human cartilage progenitor cells, etc.

For example, culture of human cartilage progenitor cell which is positive for PRRX1 protein derived from pluripotent stem cells is preferably carried out in an environment that activates Wnt signal and/or suppresses TGFβ signal.

Culture in an environment that activates Wnt signal can be carried out, for example, by culturing in the presence of an effective amount of a Wnt signal activator. Wnt signal activator is a material that enhances signal transduction (in particular, canonical Wnt pathway) mediated by Wnt. As Wnt signal activators, there may be mentioned GSK3β inhibitor, Wnt family proteins, etc. For example, as GSK3β inhibitor, there may be mentioned CHIR99021 (6-[[2-[[4-(2,4-dichlorophenyl)-5-(5-methyl-1H-imidazol-2-yl)-2-pyrimidinyl]amino]ethyl]amino]-3-pyridinecarbonitrile), XAV939 (3,5,7,8-Tetrahydro-2-[4-(trifluoromethyl)phenyl]-4H-thiopyrano[4,3-d]-pyrimidin-4-one), LiC1, etc. When CHIR99021 is used as Wnt signal activator, an amount thereof to be added may be made, for example, 0.1 to 20 µM or so, and preferably 1 to 10 µM.

Culture in an environment that suppresses TGFβ signal can be carried out, for example, by culturing in the presence of an effective amount of a TGFβ signal suppressors. The TGFβ signal suppressor is a material that is to suppress (inhibit) signal transduction mediated by TGFβ signal suppressors. As TGFβ signal suppressors, there may be mentioned A-83-01 (3-(6-methyl-2-pyridinyl)-N-phenyl-4-(4-quinolinyl)-1H-pyrazol-1-carbothioamide), SB431542 (4-[4-(1,3-benzodioxol-5-yl)-5-(2-pyridinyl)-1H-imidazol-2-yl]-benzamide), etc. When A-83-01 is used as TGFβ signal suppressor, an amount thereof to be added may be made, for example, 0.1 to 10 µM or so, and preferably 0.2 to 5 µM.

As culture, there may be exemplified by a method in which culture is carried out under the conditions of about 37°C or so and a carbon dioxide concentration of about 5% or so, but it is not limited to this. Culture under the above-mentioned conditions can be carried out, for example, using a known CO₂ incubator while controlling the temperature and the CO₂ concentration.

The term of performing culture is not particularly limited, and can be made, for example, 6 hours to 4 days or so, preferably 1 day to 3 days or so, more preferably 2 days or so (48 hours or so). If necessary, exchange of the culture medium can be carried out. The culture conditions are preferably in accordance with the usual method.

As the culture medium, it is preferable to use serum-free medium such as IMDM medium, F12 medium, or a mixed medium thereof, etc. In the state of not containing a component(s) derived from animal(s), differentiation induction and maintenance culture are possible. In addition, to the above-mentioned serum-free medium may be added various kinds of medium additives including antimicrobial agents such as streptomycin, penicillin, etc., non-essential amino acids (NEAA), ROCK inhibitor (for example, Y-27632), cell growth factors such as EGF, FGF, etc., hormones such as insulin, proteins such as transferrin, albumin, etc., lipids, polyvinyl alcohol, monothioglycerol, etc.

In this way, cell aggregates are produced from human cartilage progenitor cells. Specific embodiments are as described in Example mentioned later.

By utilizing the substrate for producing cell aggregates provided with a plurality of spots comprising a specific copolymer provided in Step (1), it is possible to form cell aggregates by adhering cells to the spots and then peeling off the same. Incidentally, the cell aggregates refer to a structural material formed as a result of aggregation of the cells, and a shape thereof is not limited to a spherical shape or a ring shape, etc. There is a merit in the point of adjusting a size of the cell aggregates according to the regulation of the adhesion area (cell aggregates having an optional size can be produced), etc., as compared with the cell aggregates produced on a conventional cell low-adhesion plate by non-adhesive culture.

### Step 4

In Step (4), the cell aggregates obtained in the above-mentioned step are cultured to produce cartilage tissue.

Culture is not particularly limited as long as it is a method capable of producing cartilage tissue from the above-mentioned cell aggregates, and is appropriately selected depending on the properties, etc., of the cell aggregates obtained from the above-mentioned human cartilage progenitor cells.

For example, culture of cell aggregates obtained from human cartilage progenitor cells which are positive for PRRX1 protein derived from pluripotent stem cells is preferably carried out by a method containing a step of culturing in an environment that activates Wnt signal.

Culture in an environment that activates Wnt signal can be carried out, for example, by culturing in the presence of an effective amount of a Wnt signal activator. Wnt signal activator is a material that enhances signal transduction (in particular, canonical Wnt pathway) mediated by Wnt. As Wnt signal activators, there may be mentioned GSK3β inhibitor, Wnt family proteins, etc. For example, as GSK3β inhibitor, there may be mentioned CHIR99021 (6-[[2-[[4-(2,4-dichlorophenyl)-5-(5-methyl-1H-imidazol-2-yl)-2-pyrimidinyl]amino]ethyl]amino]-3-pyridinecarbonitrile), XAV939 (3,5,7,8-Tetrahydro-2-[4-(trifluoromethyl)phenyl]-4H-thiopyrano[4,3-d]pyrimidin-4-one), LiC1, etc. When CHIR99021 is used as Wnt signal activator, an amount thereof to be added may be made, for example, 0.1 to 20 µM or so, and preferably 1 to 10 µM.

Also, culture is preferably carried out in multiple stages. Specifically, the process is preferably carried out in three steps of
(i) a step of subjecting to culture in the presence of a Wnt signal activator and in the presence of an FGF signal activator, then
(ii) a step of culturing in the presence of an FGF signal activator (and preferably in the absence of Wnt signal activator), then
(iii) a step of culturing preferably in the absence of a Wnt signal activator and in the absence of an FGF signal activator.

Culture in an environment that activates FGF signal can be carried out, for example, by culturing in the presence of an effective amount of an FGF signal activator. FGF signal activator is a material that enhances fibroblast growth factor (FGF) signal transduction. As FGF signal activator, there may be mentioned FGF1/aFGF, FGF2/bFGF, etc. When FGF2 is used as FGF signal activator, an amount thereof to be added may be made, for example, 0.1 to 100 ng/mL or so, and preferably 1 to 50 ng/mL.

When culture is carried out with the above-mentioned 3 steps, step (i) and step (ii) can be carried out in two-dimensional cell culture (planar culture) or three-dimensional culture. Two-dimensional cell culture can be carried out by subjecting to coating treatment of the culture apparatus to promote adhesion of cells, if necessary. Step (iii) is preferably carried out in three-dimensional culture. Incidentally, between the cultures of the respective stages, in order to reduce the influence of the previous culture environment, it is preferable to carry out the culture after appropriately subjecting to washing with PBS buffer, etc.

Culture can be carried out in an appropriate container for storing cells and culture medium. As a suitable method for subjecting to culture, there may be exemplified by a method under the conditions of about 37°C or so and a carbon dioxide concentration of about 5% or so, but it is not limited to this method. Culture under the above-mentioned conditions can be carried out, for example, while controlling temperature and CO₂ concentration using a known CO₂ incubator.

The period of subjecting to the culture in an environment that activates Wnt signal is not particularly limited to the extent that it does not impair the effect of the present invention. For example, it can be made 2 hours to 12 days or so, and 4 days to 8 days or so. In addition, when the culture is carried out with multiple stages, for example, when the culture is carried out with the above-mentioned 3 stages, the step (i) and the step (ii) can be made each 2 hours to 12 days or so, and 4 days to 8 days or so, and the step (iii) can be made 2 hours to 60 days or so, and 8 days to 54 days or so. If necessary, exchange of the culture medium can be carried out during the culturing period. The culture conditions are preferably in accordance with the usual method.

In culture, passage can be carried out if necessary. When passage is carried out, the cells are recovered before or immediately after reaching confluent state and seeded into a new culture medium.

The culture medium used for culture in step (4) is not particularly limited.
As the culture medium, serum-free medium such as IMDM medium, F12 medium, or a mixed medium thereof, etc., can be used, and a known cartilage induced medium in which L-ascorbic acid, ITS (insulin-transferrin-sodium selenite medium supplement), GDF5, and/or BMP4, etc., is/are further added to the above can be used. In addition, if necessary, known various kinds of media additive(s) including antimicrobial agents such as streptomycin, penicillin, etc., non-essential amino acids (NEAA), ROCK inhibitor (for example, Y-27632), cell growth factors such as EGF, TGFβ, etc., hormones such as insulin, proteins such as transferrin, albumin, etc., lipids, polyvinyl alcohol, monothioglycerol, etc., may be added.

Further, the culture medium used in the culture of step (4) preferably contains agar or an agar-containing medium composition.

Agar is constituted by agarose and agaropectin in which agarose is partially sulfated or substituted with a methoxy group, a pyruvate group or a carboxyl group, and there is no limitation on the ratio of these constitutional components, and it may be constituted by agarose alone. In addition, a low melting point agarose in which agarose is hydroxyethylated, a low melting point agar prepared by selecting raw seaweeds or applying hydroxyethylation, etc., and further immediately-soluble agar showing high solubility in warm water, etc., are also included in "agar " in the present invention.

In the present invention, industrially produced powdery, flake or solid agar is preferably used since they have high purity and uniform quality. Also, those having various properties and physical properties generally used in the fields of pharmaceuticals, foods, etc., can be used, and low molecular weight agar having a weight average molecular weight of 10,000 to 60,000, low strength agar having a weight average molecular weight of exceeding 60,000 and about 100,000 or less and having low gel strength, high molecular weight agar having a weight average molecular weight of 290,000 or so, etc., can be used. As such agar, commercially available products can be used, for example, "Ultra-Agar Ina", "Ultra-Agar AX-30", "Ultra-Agar AX-100", "S-6", "S-7", etc., sold by Ina Food Industry Co., Ltd., can be used.

In the present invention, it is preferable to use agar (hereinafter, in the present specification, it is sometimes referred to as "low molecular agar") which has low molecular weight than the general agar and has a weight average molecular weight of 10,000 to 60,000 and having. The weight average molecular weight of the low molecular agar is 10,000 to 60,000 as described above, preferably 20,000 to 60,000, further preferably 30,000 to 60,000, more further preferably 40,000 to 60,000, still more preferably 43,000 to 60,000, and particularly preferably 43,000 to 50,000. If agar having the weight average molecular weight of less than 10,000 is used, it may be sometimes difficult to obtain the effect of dispersing cells. On the other hand, if agar having the weight average molecular weight exceeding 60,000 is used, there is sometimes a case where dispersion of cells or tissues in the culture medium becomes non-uniform, and sufficient growth promoting effect may not be obtained.

Further, as the low molecular agar used in the present invention, it is preferable to have a narrow molecular weight distribution, and the molecular weight distribution (Mw/Mn) of agar obtained as a value by dividing the weight average molecular weight (Mw) by the number average molecular weight (Mn) is preferably 1.1 to 8.0, more preferably 1.5 to 7.0, further preferably 2.0 to 6.0, still more preferably 2.5 to 5.5, and particularly preferably 3.5 to 5.0.

Incidentally, the above-mentioned weight average molecular weight and the number average molecular weight of agar can be measured by gel permeation chromatography using high-performance liquid chromatography (HPLC).

The above-mentioned low molecular agar can be produced by known methods in which usual agar is made to reduce its molecular weight by an acid treatment, or an acid treatment of the agar at the time of extraction step from seaweeds such as Tengusa (*gelidium amansii*), Ogonori (Chinese moss), Obakusa (*Pterocladiella capillacea*), etc., or subjected to either of the above-mentioned extraction step or filtration step is carried out, etc., and the products commercially available as low molecular agar for example, the above-mentioned "Ultra Agar Ina" (available from Ina Food Industry Co., Ltd.), etc., can be also used.

Culture can be carried out by adding the above-mentioned agar as such to the culture medium used in the above-mentioned step (4) as a culture medium additive, or the culture medium composition containing the above-mentioned agar is diluted with a culture medium used in the above-mentioned step (4). As such an agar-containing culture medium composition, for example, "SphereMAX (Trademark)" (available from Nissan Chemical Corporation), etc., can be used. In addition, further specific embodiments of agar and agar-containing culture medium composition are described in WO 2016/167373.

A content of agar is preferably 0.005 (w/v) % or more and less than 2 (w/v) % based on the whole amount of the culture medium used in step (4), more preferably 0.03 (w/v) % or more and less than 2 (w/v) %, further preferably 0.03 (w/v) % to 1 (w/v) %, and still more preferably 0.03 (w/v) % to 0.1 (w/v) %. If the content of agar in the culture medium is 0.005 (w/v) % or more, the cells can grow without forming cell aggregates of excessive size, and a growth promoting effect of cells can be seen. Also, if the content of agar in the culture medium is 0.03 (w/v) % or more, uniform dispersion of cells or tissues can be obtained so that it is more preferred. On the other hand, if the content of agar in the culture medium composition is 2 (w/v) % or more, there is a case where it becomes a gel at room temperature so that it may make handling difficult in some cases.

In this way, cartilage tissue is produced. The fact that a cartilage tissue has been obtained can be confirmed by, for example, being positive for safranin O stain, alcian blue stain, safranin O stain, or toluidine blue stain, etc.

### <Use of cartilage tissue>

The present invention also relates to a method which is a method of treating human articular cartilage damage, which comprises transplanting the cartilage tissue obtained by the producing method of the present invention to a damage or defective site of articular cartilage. Here, specific embodiments and preferred embodiments of the method for producing a cartilage tissue are as mentioned above. In addition, transplantation can be carried out by inserting one or plural number of the cartilage tissues obtained by the producing method of the present invention into the damage or defective site of the articular cartilage.

### EXAMPLES

### [Preparation Example 1]

### (Preparation of substrate for producing cell aggregates)

An aqueous solution of a copolymer of 2-(N,N-dimethylamino)ethyl methacrylate and methacrylic acid (a molar ratio of 2-(N,N-dimethylaminoethyl) methacrylate based on the whole monomer is 90%) were added dropwise to the area of 1.5 cm square at the center portion of the culture surface of a culture dish (diameter: 35 mm) (Sumitomo Bakelite Co., Ltd., MS9035X) having an ability to suppress adhesion of cells using a droplet ejection system (MICROJET Corporation, BioSpot BT600) with a suitable amount in a lattice state at intervals of 300 µm in the vertical and horizontal directions, and then, the droplets were dried at room temperature for 15 minutes and fixed to prepare a substrate for producing cell aggregates having 350 independent circular spot areas with a diameter of 100 to 1,000 µm to which cells can be adhered.

### [Preparation Example 2]

### (Preparation of human cartilage progenitor cell which is positive for PRRX1 protein)

Human iPS cells (3 × 10⁴: 414C2 cell line provided by Center for iPS Cell Research and Application, Kyoto University) were suspended in 1 mL of StemFit (Registered trademark) (AJINOMOTO HEALTHY SUPPLY CO., INC.) containing 10 µM CultureSure (Registered trademark) Y-27632 (FUJIFILM Wako Pure Chemical Corporation) and 4 µL of iMatrix-511 (Nippi Inc.), and added to 35 mm culture dish. The next day, the culture medium was exchanged to new StemFit (Registered trademark) containing no Y-27632. After culture for 2 days, the cells were washed with PBS, and the culture media were exchanged with each stage to those having the following compositions to successively induce differentiation from primitive streak (mid-primitive streak), lateral plate mesoderm cells (LPM) and limb bud mesenchymal cells (LBM).

### ·Composition of culture medium for differentiation induction of primitive streak:

Serum-free medium: 50% IMDM medium (Gibco) + 50% F12 medium (Gibco) + 1 mg/ mL polyvinyl alcohol (Sigma-Aldrich) + 1% (v/v) lipid concentrate (Gibco) + 450 µM monothioglycerol (Sigma-Aldrich) + 0.7 µg/ mL insulin (Sigma-Aldrich) + 15 µg/ mL transferrin (Sigma-Aldrich) + 1% (v/v) penicillin/streptomycin (Gibco) (hereinafter, referred to as "CDM2 basal medium")
30 ng/mL activin A
40 ng/mL BMP4
6 µM CHIR99021
100 nM PIK90 (Funakoshi Co., Ltd.)
10 µM Y-27632

### ·Composition of culture medium for differentiation induction of lateral plate mesoderm (LPM):

CDM2 basal medium
1 µM A-83-01
30 ng/mL BMP4
1 µM C59
10 µM Y-27632

### ·Composition of culture medium for differentiation induction of limb bud mesenchymal cells (LBM):

CDM2 basal medium
1 µM A-83-01
0.5 µM LDN-193189
3 µM CHIR99021
150 nM Vismodegib
10 µM Y-27632

Then, the obtained LBM cells were peeled off using Accutase (Thermo Fisher Scientific), 2×10⁵ cells were suspended in the following human cartilage progenitor cell differentiation induction culture medium which contains 16 µL of iMatrix-511 (Nippi Inc.), and added to a 60 mm culture dish. Every 2 days, the culture medium was exchanged to new human cartilage progenitor cell differentiation induction culture medium to induce differentiation into human cartilage progenitor cell. The cells were similarly passaged before reaching subconfluency.

### ·Composition of culture medium for differentiation induction of human cartilage progenitor cells:

CDM2 basal medium
1 µM A-83-01
3 µM CHIR99021
20 ng/mL FGF2
20 ng/mL EGF
10 µM Y-27632

### [Example 1]

### (Production of cell aggregates)

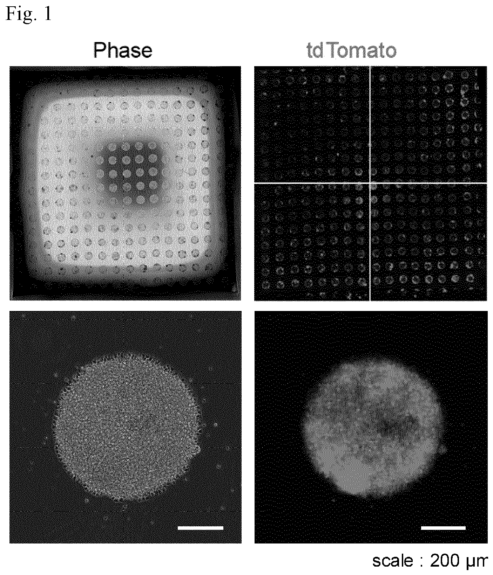

Into the culture dish obtained in Preparation Example 1 were seeded 1×10⁶ human cartilage progenitor cells (hereinafter, it is also referred to as "ExpLBM cells") obtained in Preparation Example 2 which was suspended in 1 mL of culture medium (CDM2 basal medium + 3 µM CHIR99021 + 1 µM A-83-01 + 20 ng/mL EGF + 20 ng/mL FGF), and culture was carried out at 37°C. After 4 hours, the culture medium was exchanged. Two days after culture medium exchange, cell aggregates were confirmed to be formed. The results are shown in Fig. 1.

### (Preparation of cartilage tissue)

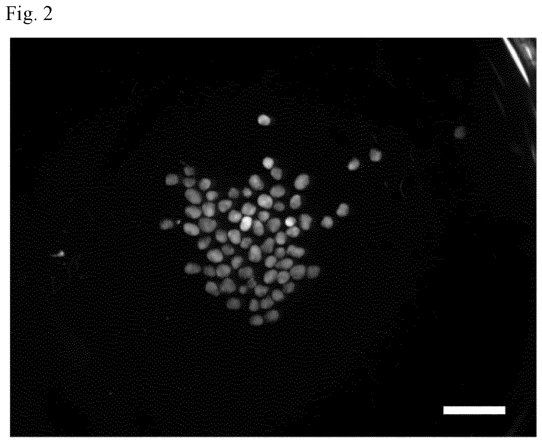

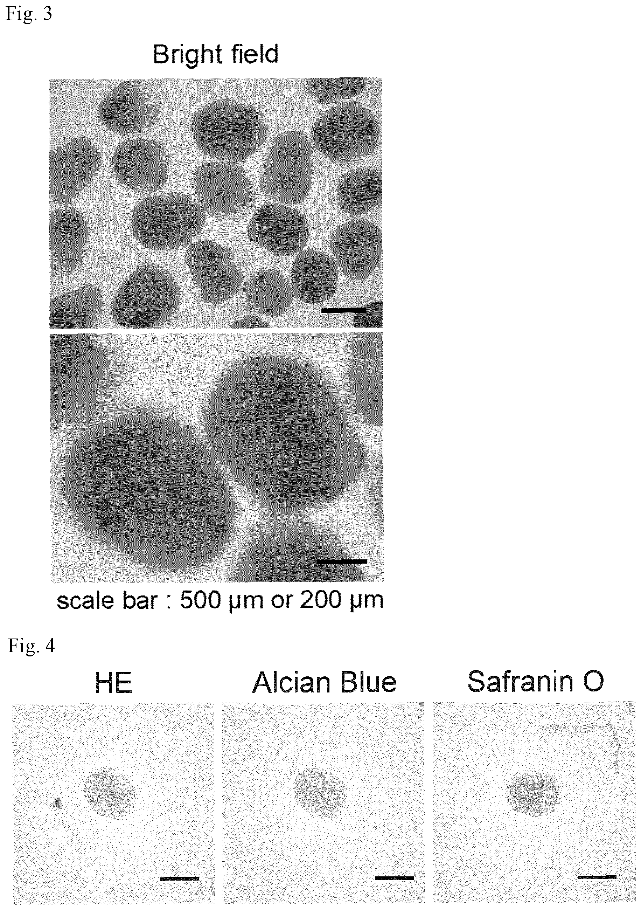

The obtained cell aggregates were peeled off and transferred to an ultra-low attachment plate (6 wells) (#3471 manufactured by Corning). The transferred cell aggregates were treated with Medium 1 for 6 days (day 0 to day 6: the culture medium composition is described below), then at the time of day 6, switched to Medium 2 and treated for 6 days (day 6 to day 12: the culture medium composition is described below), thereafter, switched to Medium 3 and culture was carried out for 42 days (day 12 to day 54: the culture medium composition is described below) to obtain cartilage tissue. The results are shown in Fig. 2 and Fig. 3. Fig. 2 is a photograph of a stereoscopic microscope, and Fig. 3 is a photograph photographed in a bright field by an inverted micrograph.

### Medium composition

Medium 1: CDM2 basal medium + 3 µM CHIR99021 + 10 ng/mL FGF2 + 50 µg mL ascorbic acid + 1×ITS + 4% low molecular agar-containing culture medium composition
Medium 2: CDM2 basal medium + 10 ng/mL FGF2 + 50 µg/mL ascorbic acid + 30 ng/mL BMP4 + 10 ng/mL TGFβ₁ + 10 ng/mL GDF5 + 1×ITS + 0.04% low molecular agar-containing culture medium composition
Medium 3: CDM2 basal medium + 50 µg/mL ascorbic acid + 30 ng/mL BMP4 + 10 ng/mL TGFβ₁ + 10 ng/mL GDF5 + 1×ITS + 0.04% low molecular agar-containing culture medium composition

The above-mentioned low molecular agar-containing culture medium composition is a low molecular agar-containing culture medium composition prepared in accordance with Text Example 10 of WO 2016/167373.

### (Observation of cartilage tissue)

The results in which the formed cartilage tissue was fixed with 4% PFA and tissue sections were prepared and stained with hematoxylin(HE), alcian blue and safranin O are each shown in Fig. 4. The staining conditions are as follows.

### ·HE staining conditions

After deparaffinization, the specimen was stained with Eosin for 1 minute, and then treated with hematoxylin for 20 minutes to carry out nuclear stain.

### ·Alcian blue staining conditions

After deparaffinization, the specimen was treated with 1% alcian blue/3% acetic acid for 20 minutes, and after washing with 3% acetic acid, treated with hematoxylin for 20 minutes to carry out nuclear stain.

### ·Safranin O staining conditions

After deparaffinization, the specimen was stained with a Weigert's iron hematoxylin solution for 10 minutes. Next, it was stained with a Fast Green solution for 5 minutes and then washing with 1% acetic acid was carried out. Next, it was stained with a 0.1% safranin O solution for 5 minutes.

### [Evaluation Example 1]

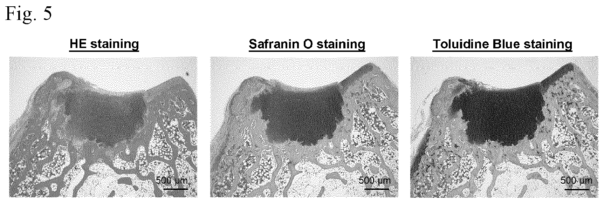

A hole (long diameter of 1.5 mm and a depth of about 1 mm) was created to the cartilage of the knee joint of SCID rats (supplied by the National BioResource Project - Rat, Tokyo University (Tokyo, Japan)) with a biopsy trepan, and the cartilage tissues (3 pieces) produced in Example 1 were pressed into the hole and transplanted. Four weeks after implantation, the tissue pieces were collected and subjected to various tissue stains (HE staining, safranin O, toluidine blue staining). The results are shown in Fig. 5. As shown in Fig. 5, it was found that engraftment was observed in the defected portion of the transplanted material, and vitreous cartilage was formed in the same region.

### UTILIZABILITY IN INDUSTY

The production method of the present invention is advantageous for the safe use of cartilage tissue for regenerative medicine since it uses quality-controlled cartilage progenitor cells as a raw material and the cells are treated to induce differentiation to obtain cartilage tissue, and since other cells than the cartilage cells are not contained in the produced cartilage tissue. Further, it is advantageous in terms of cost by carrying out production of cartilage tissue from cartilage progenitor cells under the prescribed culture conditions, it is possible to produce large cartilage tissue pieces from a small number of cells as well as efficient cell growth.

## Claims

1. A method for producing a cartilage tissue which comprises a step of providing a substrate for producing cell aggregates which is provided with a plurality of spots comprising a copolymer containing a recurring unit derived from a monomer represented by the following formula (I): 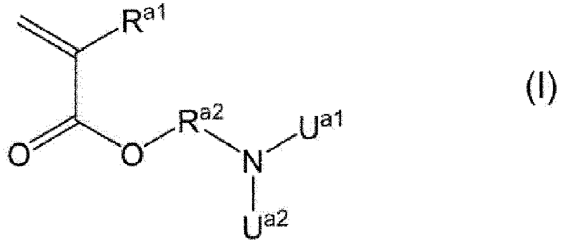 wherein
U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms,
and a recurring unit derived from a monomer represented by the following formula (II): 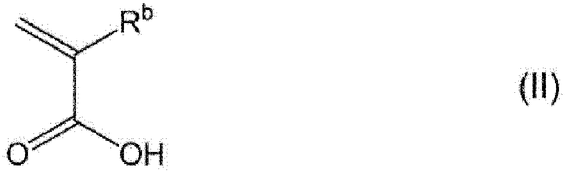 wherein
R^{b} represents a hydrogen atom or a linear or branched alkyl group having 1 to 5 carbon atoms,
on a substrate having an ability to suppress adhesion of cells; a step of seeding human cartilage progenitor cells which are positive for PRRX1 protein and derived from pluripotent stem cells on the substrate; a step of producing cell aggregates by culturing the cells; and a step of culturing the aggregates to produce a cartilage tissue.

2. The producing method according to Claim 1, wherein a molar ratio of the recurring unit derived from the monomer represented by the formula (I) based on a sum of the recurring unit derived from the monomer represented by the formula (I) and the recurring unit derived from the monomer represented by the formula (II) is 99 mol% to 51 mol%.

3. The producing method according to Claim 1 or 2, wherein the copolymer further contains a structural unit derived from a monomer represented by the following formula (III): 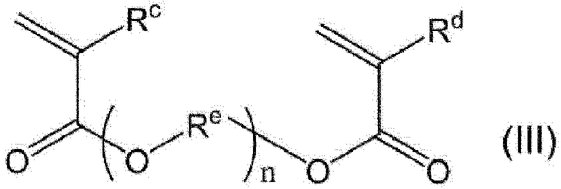 wherein
R^{c} and R^{d} each independently represent a hydrogen atom or a linear or branched alkyl group having 1 to 5 carbon atoms, R^{e} represents a linear or branched alkylene group having 1 to 5 carbon atoms and n represents a number of 1 to 50.

4. . The producing method according to any one of Claims 1 to 3, wherein the substrate having an ability to suppress adhesion of cells has a coating film containing a copolymer (P) which contains a recurring unit containing a group represented by the following formula (a) and a recurring unit containing a group represented by the following formula (b): 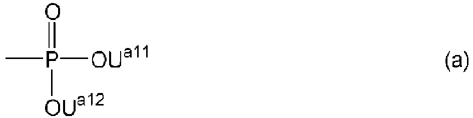 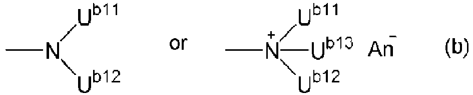 wherein
U^{a11}, U^{a12}, U^{b11}, U^{b12} and U^{b13} each independently represent a hydrogen atom or a linear or branched alkyl group having 1 to 5 carbon atoms, An⁻ represents an anion selected from the group consisting of a halide ion, an inorganic acid ion, a hydroxide ion and an isothiocyanate ion,
on at least a part of a surface thereof.

5. The producing method according to any one of Claims 1 to 4, wherein the culture in the step of producing the cartilage tissue is carried out in a culture medium containing agar.

6. The producing method according to Claim 5, wherein a weight average molecular weight of the agar is 10,000 to 60,000, and a content of the agar is 0.005 (w/v) % or more and less than 2 (w/v) % based on a whole amount of the culture medium.
